# EUROPÄISCHE PATENTSCHRIFT

(11) **EP 2 691 360 B1**
(45) Veröffentlichungstag und Bekanntmachung des Hinweises auf die Patenterteilung: **17.12.2014**
(21) Anmeldenummer: 12711159.9
(22) Anmeldetag: 27.03.2012
(51) Int. Cl.: C07C 7/20, C08F 222/06, C10L 1/14, C10M 145/16, C10L 1/196

(54) **COPOLYMERISAT UND SEINE VERWENDUNG ZUR VERBESSERUNG DER KALTFLIESSEIGENSCHAFTEN VON MITTELDESTILLAT-KRAFTSTOFFEN**
COPOLYMERS AND USE THEREOF FOR IMPROVING THE COLD FLOW PROPERTIES OF MIDDLE DISTILLATE FUELS
COPOLYMÈRES ET LEUR UTILISATION POUR AMÉLIORER DE PROPRIETES D'ECOULEMENT A FROID DES COMBUSTIBLES DISTILLATS MOYENS

(30) Priorität: 30.03.2011 EP 11160348
(43) Veröffentlichungstag der Anmeldung: 05.02.2014
(73) Patentinhaber: BASF SE, 67056 Ludwigshafen (DE)
(72) Erfinder: GARCIA CASTRO, Ivette, 67067 Ludwigshafen (DE); REBHOLZ, Uwe, 67678 Mehlingen (DE); TRÖTSCH-SCHALLER, Irene, 67281 Bissersheim (DE); BRYM, Markus, 67117 Limburgerhof (DE); STRITTMATTER, Jan, 68163 Mannheim (DE); SCHROERS, Michael, 67098 Bad Dürkheim (DE)
(86) Internationale Anmeldenummer: PCT/EP2012/055383
(87) Internationale Veröffentlichungsnummer: WO 2012/130824

(56) Entgegenhaltungen:
- EP-A1- 1 541 664
- DE-A1- 19 901 803

## Beschreibung

Die vorliegende Erfindung betrifft ein neues Copolymerisat auf Basis von (i) ethylenisch α,β-ungesättigten Dicarbonsäuren oder Derivaten hiervon und (ii) ethylenisch ungesättigten Carbonsäureestern, bei denen die ethylenische Doppelbindung nicht in Konjugation mit dem Carboxyl-Kohlenstoffatom steht, wobei die aus den ethylenisch α,β-ungesättigten Dicarbonsäuren stammenden Carboxylgruppen teilweise oder vollständig mit einem langkettigen Alkohol verestert sind.

Weiterhin betrifft die vorliegende Erfindung ein Verfahren zur Herstellung dieses Copolymerisates durch radikalische Copolymerisation.

Weiterhin betrifft die vorliegende Erfindung die Verwendung dieses Copolymerisates zur Verbesserung der Kaltfließeigenschaften von Mitteldestillat-Kraftstoffen, insbesondere zur Erniedrigung deren CP-Wertes, sowie Mitteldestillat-Kraftstoffe mit einem Gehalt and diesem Copolymerisat selbst.

Mitteldestillat-Kraftstoffe aus fossilem Ursprung, insbesondere Gasöle, Dieselöle oder leichte Heizöle, die aus Erdöl gewonnen werden, haben je nach Herkunft des Rohöls unterschiedliche Gehalte an Paraffinen. Bei tiefen Temperaturen kommt es am Trübungspunkt oder Cloud Point ("CP") zur Ausscheidung fester Paraffine. Bei weiterer Abkühlung bilden die plättchenförmigen n-Paraffinkristalle eine Art von "Kartenhausstruktur" und der Mitteldestillat-Kraftstoff stockt, obwohl sein überwiegender Teil noch flüssig ist. Durch die ausgefallenen n-Paraffine im Temperaturbereich zwischen Trübungspunkt (Cloud Point) und Pour Point ("PP") wird die Fließfähigkeit der Mitteldestillat-Kraftstoffe erheblich beeinträchtigt; die Paraffine verstopfen Filter und verursachen eine ungleichmäßige oder völlig unterbrochene Kraftstoffzufuhr zu den Verbrennungsaggregaten. Ähnliche Störungen treten bei leichten Heizölen auf.

Es ist seit langem bekannt, dass durch geeignete Zusätze das Kristallwachstum der n-Paraffine in Mitteldestillat-Kraftstoffen modifiziert werden kann. Gut wirksame Additive verhindern, dass Mitteldestillat-Kraftstoffe bei Temperaturen wenige Grade Celsius unterhalb der Temperatur, bei welcher die ersten Paraffinkristalle auskristallisieren, bereits fest werden. Statt dessen werden feine, gut kristallisierende, separate Paraffinkristalle gebildet, welche auch bei weiterer Absenkung der Temperatur Filter in Kraftfahrzeugen und Heizungsanlagen passieren oder zumindest einen für den flüssigen Teil der Mitteldestillate durchlässigen Filterkuchen bilden, so dass ein störungsfreier Betrieb sichergestellt ist. Die Wirksamkeit der Fließverbesserer wird üblicherweise nach der europäischen Norm EN 116 indirekt durch Messung des Cold Filter Plugging Point ("CFPP") ausgedrückt. Als derartige Kaltfließverbesserer oder Middle Distillate Flow Improvers ("MDFI") werden beispielsweise schon seit langem Ethylen-Vinylcarboxylat-Copolymere wie Ethylen-Vinylacetat-Copolymere ("EVA") eingesetzt.

Ein Nachteil dieser Additive liegt darin, dass die derart modifizierten Paraffinkristalle aufgrund ihrer gegenüber dem flüssigen Teil höheren Dichte dazu neigen, sich beim Lagern des Mitteldestillat-Kraftstoffes mehr und mehr am Boden des Behälters abzusetzen. Dadurch bildet sich im oberen Behälterteil eine homogene paraffinarme Phase und am Boden eine zweiphasige paraffinreiche Schicht. Da sowohl in den Fahrzeugtanks als auch in Lager- oder Liefertanks der Mineralölhändler der Abzug des Kraftstoffes meist wenig oberhalb des Behälterbodens erfolgt, besteht die Gefahr, dass die hohe Konzentration an festen Paraffinen zu Verstopfungen von Filtern und Dosiereinrichtungen führt. Diese Gefahr wird um so größer, je weiter die Lagertemperatur die Ausscheidungstemperatur der Paraffine unterschreitet, da die ausgeschiedene Paraffinmenge mit sinkender Temperatur zunimmt. Insbesondere verstärken auch Anteile an Biodiesel diese unerwünschte Neigung des Mitteldestillat-Kraftstoffes zur Paraffinsedimentation. Durch den zusätzlichen Einsatz von Paraffindispergatoren oder Wax Anti-Settling Additiven ("WASA") können die geschilderten Probleme verringert werden.

Im Zuge abnehmender Welterdölreserven und der Diskussion um die die Umwelt beeinträchtigenden Konsequenzen des Verbrauchs fossiler und mineralischer Brennstoffe steigt das Interesse an alternativen, auf nachwachsenden Rohstoffen basierenden Energiequellen. Dazu gehören insbesondere native Öle und Fette pflanzlichen oder tierischen Ursprungs. Dies sind insbesondere Triglyceride von Fettsäuren mit 10 bis 24 Kohlenstoffatomen, die zu Niedrigalkylestern wie Methylestern umgesetzt werden. Diese Ester werden allgemein auch als "FAME" (Fatty Acid Methyl Ester) bezeichnet.

Wie bei Mitteldestillaten aus fossilem Ursprung fallen beim Abkühlen solcher FAME Kristalle aus, welche ebenfalls Kraftfahrzeugfilter und Dosiereinrichtungen zusetzen können. Diese Kristalle bestehen jedoch nicht aus n-Paraffinen sondern aus Fettsäureestern, trotzdem lassen sich Kraftstoffe auf Basis von FAME mit den gleichen Kenngrößen wie bei den Mitteldestillaten aus fossilem Ursprung (CP, PP, CFPP) charakterisieren.

Die genannten FAME sowie Mischungen dieser FAME mit Mitteldestillaten besitzen in der Regel ein schlechteres Kälteverhalten als Mitteldestillate aus fossilem Ursprung alleine. Die Zugabe der FAME erhöht bei Mischungen mit Mitteldestillaten aus fossilem Ursprung die Tendenz zur Bildung von Paraffinsedimenten. Insbesondere jedoch weisen die genannten FAME, wenn sie als Biobrennstofföle Mitteldestillate aus fossilem Ursprung teilweise oder vollständig ersetzen sollen, zu hohe CFPP-Werte und vor allem zu hohe PP-Werte auf, so dass sie nicht problemlos als Kraftstoff oder Heizöl entsprechend den geltenden länder- und regionalspezifischen Anforderungen eingesetzt werden können. Auch beeinflusst die Zunahme der Viskosität bei Abkühlung die Kälteeigenschaft bei FAME stärker als bei Mitteldestillaten aus fossilem Ursprung.

Es sind bereits Additive vorgeschlagen worden, die die Kälteeigenschaften, beispielsweise den CP-Wert, von Mitteldestillat-Kraftstoffen verbessern sollen. So werden in der EP 1 746 147 A1 Copolymerisate aus Maleinsäure-mono-C₈- bis C₃₀-alkylestern, Maleinsäureanhydrid und C₈- bis C₃₀-Alkenen für diese Verwendung in Heizölen und Dieselkraftstoffen, die auch Biobrennstofföle in Mengen bis zu 30 Gew.-% enthalten können, beschrieben.

Es bestand die Aufgabe, Produkte zur Verfügung zu stellen, welche ein verbessertes Kälteverhalten bei Mitteldestillat-Kraftstoffen, insbesondere bei solchen auf Basis von Biobrennstoffölen ("Biodiesel"), welche auf Fettsäureestern (FAME) basiert, bewirken. Insbesondere sollte der Pour Point (PP-Wert) für solche Kraftstoffe effektiv abgesenkt werden.

Die Aufgabe wird erfindungsgemäß durch ein Copolymerisat gelöst, welches aufgebaut ist aus
(i) 10 bis 90 Mol-% Wiederholungseinheiten der Struktur W1 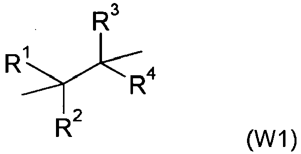 in der die Variablen R¹ und R² Wasserstoff, C₁- bis C₄-Alkyl oder Carboxylester-Gruppierungen der Formel -COOR⁹ bedeuten, wobei R⁹ für einen C₆- bis C₃₀-Hydrocarbylrest steht und wobei eine der Variablen R¹ oder R² Wasserstoff oder C₁- bis C₄-Alkyl und die andere eine Carboxylester-Gruppierung der Formel -COOR⁹ bedeutet, und
   in der Variablen R³ und R⁴ Wasserstoff, C₁- bis C₄-Alkyl, Carboxylester-Gruppierungen der Formel -COOR⁹, wobei R⁹ für einen C₆- bis C₃₀-Hydrocarbylrest steht, oder Carboxylgruppen, welche auch in Form ihrer Alkalimetall-, Erdalkalimetall- oder Ammoniumsalze vorliegen können, bedeuten, wobei eine der Variablen R³ oder R⁴ Wasserstoff oder C₁- bis C₄-Alkyl und die andere eine Carboxylester-Gruppierung der Formel -COOR⁹ und/oder eine Carboxylgruppe, welche auch in Form ihres Alkalimetall-, Erdalkalimetall- oder Ammoniumsalzes vorliegen kann, bedeutet, und
(ii) 90 bis 10 Mol-% Wiederholungseinheiten der Struktur W2 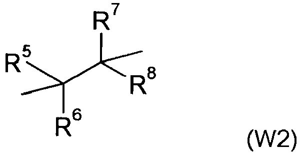 in der die Variable R⁵ den Rest eines Carbonsäureesters der Formel

   -A-CO-O-R¹⁰

   bedeutet, wobei die Variable A für eine C₁- bis C₂₀-Alkylengruppe steht und die Variable R¹⁰ einen C₁- bis C₃₀-Hydrocarbylrest bezeichnet, und
   in der die Variablen R⁶, R⁷ und R⁸ unabhängig voneinander für Wasserstoff oder C₁- bis C₈-Alkyl stehen,
wobei die Summe der Wiederholungseinheiten W1 und W2 100 Mol-% ergibt.

Im Rahmen der vorliegenden Erfindung gelten für generisch definierte Reste folgende Definitionen:
Hydrocarbylreste sind Reste aus linearen oder verweigten Kohlenwasserstoffketten, die auch in geringem Umfang Heteroatome wie Sauerstoff, Stickstoff oder Halogenatome, beispielsweise Chlor, und/oder nicht-protische funktionelle Gruppen wie beispielsweise Carboxylestergruppen, Cyanogruppen oder Nitrogruppen enthalten können, ohne den überwiegend hydrophoben Kohlenwasserstoff-Charakter dieser Reste wesentlich zu beeinflussen, und in der Regel keine Kohlenstoff-Kohlenstoff-Doppel- oder -Dreifachbindungen und auch keine sonstigen Ungesättigtheiten, welche Bildung von Copolymerisaten aus (i) und (ii) stören könnten, aufweisen. Bevorzugt werden als C₁- bis C₃₀-Hydrocarbylreste jedoch reine Cycloalkylreste, welche auch Alkylseitenketten tragen können, und/oder insbesondere reine lineare oder verzweigte Alkylreste mit der jeweils entsprechend gleichen Gesamtanzahl von Kohlenstoffatomen.

Beispiele für derartige C₁- bis C₄- bzw. C₁- bis C₈- bzw. C₁- bis C₃₀- bzw. C₆- bis C₃₀- bzw. C₈- bis C₁₆- bzw. C₁₀- bis C₁₄-Alkyl- oder -Cycloalkylreste sind Methyl, Ethyl, n-Propyl, Isopropyl, n-Butyl, 2-Butyl, Isobutyl, tert.-Butyl, Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl, n-Octyl, 2-Ethylhexyl, n-Nonyl, iso-Nonyl, n-Decyl, 2-Propylheptyl, n-Undecyl, n-Dodecyl, n-Tridecyl, iso-Tridecyl, n-Tetradecyl, n-Hexadecyl, n-Octadecyl und n-Eicosyl sowie als entsprechende Cycloalkylreste, die auch Alkylseitenketten tragen können, Cyclopentyl, 2- oder 3-Methylcyclopentyl, 2,3-, 2,4- oder 2,5-Dimethylcyclopentyl, Cyclohexyl, 2-, 3- oder 4-Methylcyclohexyl, 2,3-, 2,4-, 2,5-, 2,6-, 3,4-, 3,5- oder 3,6-Dimethylcyclohexyl, Cylcoheptyl, 2-, 3- oder 4-Methylcycloheptyl, Cyclooctyl, 2-, 3-, 4- oder 5-Methylcyclooctyl. Hier in Betracht kommende stabile Cycloalkylreste tragen üblicherweise mindestens 4 Kohlenstoffatome im Ring.

Als C₁- bis C₂₀- bzw. lineare C₄- bis C₁₂-Alkylengruppen für die Variable A eignen sich im Prinzip alle divalenten linearen oder verzweigten gesättigten aliphatischen Kohlenwasserstoffkörper, bevorzugt werden jedoch Polyalkylengruppen der Formel -(CH₂)ₘ-, in der m für eine Zahl von 1 bis 20, insbesondere 2 bis 16, vor allem 4 bis 12, ganz besonders bevorzugt 6 bis 10, beispielsweise 7, 8 oder 9, steht. Bei verzweigten Alkylengruppen eignen sich neben den α,ω-verknüpfenden Kohlenwasserstoffkörpern auch nicht lineare Brückenglieder wie 1,1-Ethylen, 1,1-Propylen, 2,2-Propylen, 1,2-Propylen, 2-Methyl-1,4-butylen, 3-Methyl-1,5-pentylen oder 2-Ethyl-1,6-hexylen.

Das erfindungsgemäße Copolymerisat kann im Prinzip statistisch, blockartig oder alternierend aufgebaut sein. Ein alternierender Aufbau wird bevorzugt. Demgemäß ist das erfindungsgemäße Copolymerisat vorzugsweise aus 25 bis 75 Mol-%, insbesondere 45 bis 55 Mol-%, vor allem 49 bis 51 Mol-% Wiederholungseinheiten der Struktur W1 und 75 bis 25 Mol-%, insbesondere 55 bis 45 Mol-%, vor allem 51 bis 49 Mol-% Wiederholungseinheiten der Struktur W2 aufgebaut.

In einer bevorzugten Ausführungsform haben die Variablen in der Wiederholungseinheit W1 die folgenden Bedeutungen:
- R¹: Wasserstoff,
- R²: eine Carboxylester-Gruppierung der Formel -COOR⁹, wobei R⁹ für einen C₈- bis C₁₆-, insbesondere einen C₁₀- bis C₁₄-Hydrocarbylrest, steht,
- R³: Wasserstoff,
- R⁴: eine Carboxylester-Gruppierung der Formel -COOR⁹, wobei R⁹ für einen C₈- bis C₁₆-, insbesondere einen C₁₀- bis C₁₄-Hydrocarbylrest, steht, und/oder eine Carboxylgruppe, welche auch in Form ihres Alkalimetall-, Erdalkalimetall- oder Ammoniumsalzes vorliegen kann

In einer weiteren bevorzugten Ausführungsform haben die Variablen in der Wiederholungseinheit W2 die folgenden Bedeutungen:
- R⁵: den Rest eines Carbonsäureesters der Formel -A-CO-O-R¹⁰, wobei A eine lineare C₄- bis C₁₂-Alkylengruppe und R¹⁰ einen C₁- bis C₄-Alkylrest bezeichnet,
- R⁶, R⁷ und R⁸: Wasserstoff

Sollten Carboxylgruppen in der Wiederholungseinheit W1 in Form ihrer Alkalimetall-, Erdalkalimetall- oder Ammoniumsalze vorliegen, so sind hier beispielsweise die Lithium-, Natrium-, Kalium-, Magnesium- oder Calciumsalze gemeint. Bei Ammoniumsalzen kann das Ammoniumkation am Stickstoff beispielsweise bis zu 4 gleiche oder verschiedene Alkylsubstituenten wie Methyl, Ethyl, n-Propyl oder n-Butyl tragen.

Das erfindungsgemäße Copolymerisat lässt sich vorteilhafterweise nach bekannten und üblichen radikalischen Polymerisationstechniken herstellen. Daher ist auch Gegenstand der vorliegenden Erfindung ein Copolymerisat, welches durch radikalische Copolymerisation von
(i) 10 bis 90 Mol-%, vorzugsweise 25 bis 75 Mol-%, insbesondere 45 bis 55 Mol-%, vor allem 49 bis 51 Mol-% Monomereinheiten der Struktur M1 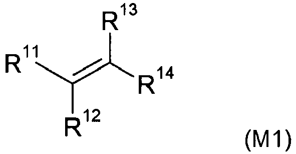 in der die Variablen R¹¹, R¹², R¹³ und R¹⁴ unabhängig voneinander Wasserstoff, C₁- bis C₄-Alkyl, Carboxylgruppen, Carboxylester-Gruppierungen der Formel -COOR¹⁵, wobei R¹⁵ für einen C₁- bis C₄-Alkylrest steht, oder Carboxylhalogenid-Gruppierungen der Formel -COX, wobei X für Fluor, Chlor, Brom oder Jod steht, bezeichnen, mit der Maßgabe, dass M1 zwei vicinal ständige derartige Carboxylgruppen und/oder Carboxylester-Gruppierungen in cis- oder trans-Stellung zueinander enthält, wobei bei vicinal ständigen Carboxylgruppen in cis-Stellung diese auch in Form ihres cyclischen Anhydrids vorliegen können, und
(ii) 90 bis 10 Mol-%, vorzugsweise 75 bis 25 Mol-%, insbesondere 55 bis 45 Mol-%, vor allem 51 bis 49 Mol-% Monomereinheiten der Struktur M2 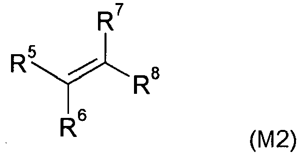 in der die Variable R⁵ den Rest eines Carbonsäureesters der Formel

   -A-CO-O-R¹⁰

   bedeutet, wobei die Variable A für eine C₁- bis C₂₀-Alkylengruppe, insbesondere eine lineare C₄- bis C₁₂-Alkylengruppe, steht und die Variable R¹⁰ einen C₁- bis C₃₀-Hydrocarbylrest, insbesondere einen C₁- bis C₈-Hydrocarbylrest, vor allem einen C₁- bis C₄-Alkylrest, bezeichnet, und
   in der die Variablen R⁶, R⁷ und R⁸ unabhängig voneinander für Wasserstoff oder C₁- bis C₈-Alkyl stehen,
wobei die Summe der Monomereinheiten M1 und M2 100 Mol-% ergibt,
und anschließende polymeranaloge Umsetzung des gebildeten Produktes mit mindestens 1 bis 2 Mol eines C₆- bis C₃₀-Hydrocarbylalkohols pro Mol eingesetztem Monomer M1 erhältlich ist.

Gegenstand der vorliegenden Erfindung ist auch ein Verfahren zur Herstellung des erfindungsgemäßen Copolymerisates, welches dadurch gekennzeichnet ist, dass man
(i) 10 bis 90 Mol-%, vorzugsweise 25 bis 75 Mol-%, insbesondere 45 bis 55 Mol-%, vor allem 49 bis 51 Mol-% Monomereinheiten der Struktur M1 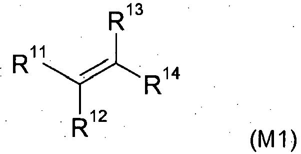 in der die Variablen R¹¹, R¹², R¹³ und R¹⁴ unabhängig voneinander Wasserstoff, C₁- bis C₄-Alkyl, Carboxylgruppen, Carboxylester-Gruppierungen der Formel -COOR¹⁵, wobei R¹⁵ für einen C₁- bis C₄-Alkylrest steht, oder Carboxylhalogenid-Gruppierungen der Formel -COX, wobei X für Fluor, Chlor, Brom oder Jod steht, bezeichnen, mit der Maßgabe, dass M1 zwei vicinal ständige derartige Carboxylgruppen und/oder Carboxylester-Gruppierungen in cis- oder trans-Stellung zueinander enthält, wobei bei vicinal ständigen Carboxylgruppen in cis-Stellung diese auch in Form ihres cyclischen Anhydrids vorliegen können, und
(ii) 90 bis 10 Mol-%, vorzugsweise 75 bis 25 Mol-%, insbesondere 55 bis 45 Mol-%, vor allem 51 bis 49 Mol-% Monomereinheiten der Struktur M2 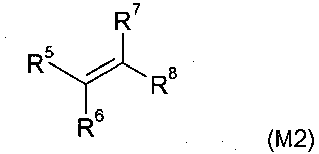 in der die Variable R⁵ den Rest eines Carbonsäureesters der Formel

   -A-CO-O-R¹⁰

   eine lineare C₄- bis C₁₂-Alkylengruppe, steht und die Variable R¹⁰ einen C₁- bis C₃₀-Hydrocarbylrest, insbesondere einen C₁- bis C₈-Hydrocarbylrest, vor allem einen C₁- bis C₄-Alkylrest, bezeichnet, und
   in der die Variablen R⁶, R⁷ und R⁸ unabhängig voneinander für Wasserstoff oder C₁- bis C₈-Alkyl stehen,
wobei die Summe der Monomereinheiten M1 und M2 100 Mol-% ergibt,
radikalisch miteinander copolymerisiert und anschließend das gebildete Produkt polymeranalog mit mindestens 1 bis 2 Mol eines C₆- bis C₃₀-Hydrocarbylalkohols pro Mol eingesetztem Monomer M1 umsetzt.

Die Monomerkomponenten (i) stellen ethylenisch α,β-ungesättigten Dicarbonsäuren oder Derivaten hiervon wie Ester, cyclische Anhydride oder Carbonsäurehalogenide wie Carbonsäurechloride dar. In einer bevorzugten Ausführungsform setzt man bei der Copolymerisation von M1 und M2, welche zweckmäßigerweise unter Verwendung von radikalisch zerfallenden Initiatoren durchgeführt wird, Maleinsäure, Maleinsäureanhydrid, Maleinsäuremono- oder -dimethylester, Maleinsäuremono- oder -diethylester, Fumarsäure, Fumarsäuremono- oder -dimethylester oder Fumarsäuremono- oder -diethylester als Momomereinheiten M1 ein. Weiterhin eignen sich aber als Monomereinheiten M1 beispielsweise auch gut 2-Methylmaleinsäure, 2-Methylmaleinsäureanhydrid, 2,3-Dimethylmaleinsäure, 2,3-Dimethylmaleinsäureanhydrid, 2-Methylfumarsäure, 2,3-Dimethylfumarsäure sowie die Mono- und Dimethyl- und Mono- und Diethylester dieser Dicarbonsäuren.

Die Monomerkomponenten (ii) stellen ethylenisch ungesättigten Carbonsäureestern, bei denen die ethylenische Doppelbindung nicht in Konjugation mit dem Carboxyl-Kohlenstoffatom steht, dar. Vorzugsweise setzt man hierbei solche Monomereinheiten M2 ein, in denen die Variablen R⁶, R⁷ und R⁸ für Wasserstoff stehen, die Variable R¹⁰ einen C₁- bis C₄-Alkylrest, insbesondere Methyl, Ethyl, n-Propyl oder n-Butyl, bezeichnet und die Variable A für eine lineare C₄- bis C₁₂-Alkylengruppe, insbesondere eine lineare C₆- bis C₁₀-Alkylengruppe, vor allem eine lineare C₇-, C₈- oder C₉-Alkylengruppe, steht. Beispiele für Monomereinheiten M2 sind:
But-3-ensäure-methyl-, ethyl-, n-propyl und n-butylester,
Pent-3-ensäure-methyl-, ethyl-, n-propyl und n-butylester,
   Pent-4-ensäure-methyl-, ethyl-, n-propyl und n-butylester,
Hex-3-ensäure-methyl-, ethyl-, n-propyl und n-butylester,
   Hex-4-ensäure-methyl-, ethyl-, n-propyl und n-butylester,
   Hex-5-ensäure-methyl-, ethyl-, n-propyl und n-butylester,
Hept-3-ensäure-methyl-, ethyl-, n-propyl und n-butylester,
   Hept-4-ensäure-methyl-, ethyl-, n-propyl und n-butylester,
   Hept-5-ensäure-methyl-, ethyl-, n-propyl und n-butylester,
   Hept-6-ensäure-methyl-, ethyl-, n-propyl und n-butylester,
Oct-3-ensäure-methyl-, ethyl-, n-propyl und n-butylester,
   Oct-4-ensäure-methyl-, ethyl-, n-propyl und n-butylester,
   Oct-5-ensäure-methyl-, ethyl-, n-propyl und n-butylester,
   Oct-6-ensäure-methyl-, ethyl-, n-propyl und n-butylester,
   Oct-7-ensäure-methyl-, ethyl-, n-propyl und n-butylester,
Non-3-ensäure-methyl-, ethyl-, n-propyl und n-butylester,
   Non-4-ensäure-methyl-, ethyl-, n-propyl und n-butylester,
   Non-5-ensäure-methyl-, ethyl-, n-propyl und n-butylester,
   Non-6-ensäure-methyl-, ethyl-, n-propyl und n-butylester,
   Non-7-ensäure-methyl-, ethyl-, n-propyl und n-butylester,
   Non-8-ensäure-methyl-, ethyl-, n-propyl und n-butylester,
Dec-3-ensäure-methyl-, ethyl-, n-propyl und n-butylester,
   Dec-4-ensäure-methyl-, ethyl-, n-propyl und n-butylester,
   Dec-5-ensäure-methyl-, ethyl-, n-propyl und n-butylester,
   Dec-6-ensäure-methyl-, ethyl-, n-propyl und n-butylester,
   Dec-7-ensäure-methyl-, ethyl-, n-propyl und n-butylester,
   Dec-8-ensäure-methyl-, ethyl-, n-propyl und n-butylester,
   Dec-9-ensäure-methyl-, ethyl-, n-propyl und n-butylester,
Undec-3-ensäure-methyl-, ethyl-, n-propyl und n-butylester,
   Undec-4-ensäure-methyl-, ethyl-, n-propyl und n-butylester,
   Undec-5-ensäure-methyl-, ethyl-, n-propyl und n-butylester,
   Undec-6-ensäure-methyl-, ethyl-, n-propyl und n-butylester,
   Undec-7-ensäure-methyl-, ethyl-, n-propyl und n-butylester,
   Undec-8-ensäure-methyl-, ethyl-, n-propyl und n-butylester,
   Undec-9-ensäure-methyl-, ethyl-, n-propyl und n-butylester,
   Undec-10-ensäure-methyl-, ethyl-, n-propyl und n-butylester,
Dodec-3-ensäure-methyl-, ethyl-, n-propyl und n-butylester,
   Dodec-4-ensäure-methyl-, ethyl-, n-propyl und n-butylester,
   Dodec-5-ensäure-methyl-, ethyl-, n-propyl und n-butylester,
   Dodec-6-ensäure-methyl-, ethyl-, n-propyl und n-butylester,
   Dodec-7-ensäure-methyl-, ethyl-, n-propyl und n-butylester,
   Dodec-8-ensäure-methyl-, ethyl-, n-propyl und n-butylester,
   Dodec-9-ensäure-methyl-, ethyl-, n-propyl und n-butylester,
   Dodec-10-ensäure-methyl-, ethyl-, n-propyl und n-butylester,
   Dodec-11-ensäure-methyl-, ethyl-, n-propyl und n-butylester,
Tridec-3-ensäure-methyl-, ethyl-, n-propyl und n-butylester,
   Tridec-4-ensäure-methyl-, ethyl-, n-propyl und n-butylester,
   Tridec-5-ensäure-methyl-, ethyl-, n-propyl und n-butylester,
   Tridec-6-ensäure-methyl-, ethyl-, n-propyl und n-butylester,
   Tridec-7-ensäure-methyl-, ethyl-, n-propyl und n-butylester,
   Tridec-8-ensäure-methyl-, ethyl-, n-propyl und n-butylester,
   Tridec-9-ensäure-methyl-, ethyl-, n-propyl und n-butylester,
   Tridec-10-ensäure-methyl-, ethyl-, n-propyl und n-butylester,
   Tridec-11-ensäure-methyl-, ethyl-, n-propyl und n-butylester,
   Tridec-12-ensäure-methyl-, ethyl-, n-propyl und n-butylester,
Tetradec-3-ensäure-methyl-, ethyl-, n-propyl und n-butylester,
   Tetradec-4-ensäure-methyl-, ethyl-, n-propyl und n-butylester,
   Tetradec-5-ensäure-methyl-, ethyl-, n-propyl und n-butylester,
   Tetradec-6-ensäure-methyl-, ethyl-, n-propyl und n-butylester,
   Tetradec-7-ensäure-methyl-, ethyl-, n-propyl und n-butylester,
   Tetradec-8-ensäure-methyl-, ethyl-, n-propyl und n-butylester,
   Tetradec-9-ensäure-methyl-, ethyl-, n-propyl und n-butylester,
   Tetradec-10-ensäure-methyl-, ethyl-, n-propyl und n-butylester,
   Tetradec-11-ensäure-methyl-, ethyl-, n-propyl und n-butylester,
   Tetradec-12-ensäure-methyl-, ethyl-, n-propyl und n-butylester,
   Tetradec-13-ensäure-methyl-, ethyl-, n-propyl und n-butylester,
Pentadec-3-ensäure-methyl-, ethyl-, n-propyl und n-butylester,
   Pentadec-4-ensäure-methyl-, ethyl-, n-propyl und n-butylester,
   Pentadec-5-ensäure-methyl-, ethyl-, n-propyl und n-butylester,
   Pentadec-6-ensäure-methyl-, ethyl-, n-propyl und n-butylester,
   Pentadec-7-ensäure-methyl-, ethyl-, n-propyl und n-butylester,
   Pentadec-8-ensäure-methyl-, ethyl-, n-propyl und n-butylester,
   Pentadec-9-ensäure-methyl-, ethyl-, n-propyl und n-butylester,
   Pentadec-10-ensäure-methyl-, ethyl-, n-propyl und n-butylester,
   Pentadec-11-ensäure-methyl-, ethyl-, n-propyl und n-butylester,
   Pentadec-12-ensäure-methyl-, ethyl-, n-propyl und n-butylester,
   Pentadec-13-ensäure-methyl-, ethyl-, n-propyl und n-butylester,
   Pentadec-14-ensäure-methyl-, ethyl-, n-propyl und n-butylester

Die ethylenische Doppelbindung in allen den oben genannten Monomerkomponenten M2 kann cis- oder trans-konfiguriert sein.

Man kann die beiden Monomerkomponenten (i) und (ii) in der ersten Herstellungsstufe in Substanz, in Suspension oder vorzugsweise in Lösung polymerisieren. Man kann hierbei für beide Monomerkomponenten (i) und (ii) jeweils eine einzelne Monomerspezies oder Mischung aus mehreren solcher Monomerspezies einsetzen. Man führt die Polymerisationsreaktion der Regel bei Normaldruck und unter einem Schutzgas wie Stickstoff durch, kann aber auch bei erhöhten Drücken, beispielsweise in einem Autoklaven, arbeitet. Die Polymerisationstemperaturen liegen in der Regel bei 50 bis 250°C, insbesondere bei 90 bis 210°C, vor allem bei 120 bis 180°C, typischerweise bei 140 bis 160°C. Als Polymerisationsreaktor eignen sich im Prinzip alle üblichen kontinuierlich oder diskontinuierlich betriebenen Apparaturen wie beispielsweise Rührkessel, Rührkesselkaskade, Rohrreaktor oder Schlaufenreaktor.

Üblicherweise wird die Polymerisation durch radikalisch zerfallende Initiatoren gestartet, hierzu eignen sich Luft oder Sauerstoff oder organischen Peroxiden und/oder Hydroperoxiden sowie organische Azoverbindungen. Als organische Peroxide bzw. Hydroperoxide kommen beispielsweise Diisopropylbenzolhydroperoxid, Cumolhydroperoxid, Methylisobutylketonperoxid, Di-tert.-butylperoxid und tert.-Butylperiisononat in Betracht. Als organische Azoverbindung ist beispielsweise Azobisisobutyronitril ("AIBN") geeignet. Weiterhin können geeignete Regler wie aliphatische Aldehyde oder Ketone oder auch Wasserstoff bei der Polymerisation mitverwendet werden.

Falls Lösungs- oder Suspensionsmittel bei der Polymerisation mitverwendet werden, kommen hierfür die üblichen hochsiedenden inerten Flüssigkeiten wie aromatische Kohlenwassestoffe, z. B. Toluol, Xylole oder entsprechende technische Kohlenwasserstoffgemische wie Solvesso® oder Solvent Naphtha, in Betracht.

Als C₆- bis C₃₀-Hydrocarbylalkohole kommen für die polymeranaloge Umsetzung des wie oben beschrieben hergestellten Polymerisationsproduktes in der zweiten Herstellungsstufe solche Alkohole in Betracht, die die oben genannten Hydrocarbylreste, insbesondere Alkyl- oder Cycloalkylreste, tragen. Bevorzugt werden hierbei allerdings verzweigte oder insbesondere lineare primäre C₈- bis C₁₆-Hydrocarbylalkohole, vor allem verzweigte oder insbesondere lineare primäre C₁₀- bis C₁₄-Hydrocarbylalkohole. Typische Beispiele für solche Hydrocarbylalkohole sind 2-Ethylhexanol, n-Octanol, n-Nonanol, n-Decanol, 2-Propylheptanol, n-Undecanol, n-Dodecanol, n-Tridecanol und iso-Tridecanol. Es können auch technische Gemische solcher mittelkettiger aliphatischer Alkohole eingesetzt werden.

Die Umsetzung des Polymerisationsproduktes mit dem C₆- bis C₃₀-Hydrocarbylalkohol oder mit einem Gemisch solcher Alkohole erfolgt in der Regel durch Erhitzen bei Normaldruck und typischerweise unter einem Schutzgas wie Stickstoff auf Temperaturen im Bereich von 50 bis 200°C, insbesondere von 90 bis 180°C, vor allem von 120 bis 170°C, typischerweise von 140 bis 160°C. Man kann hierbei Säuren oder Basen als Veresterungskatalysatoren mitverwenden. Wenn die Veresterung vollständig ist oder den gewünschten Umsatzgrad erreicht hat, arbeitet man wie üblich auf. Liegen die zu veresternden Carboxylgruppe als Ester von Niedrigalkoholen, d. h. als C₁- bis C₄-Alkylester vor, findet eine Umesterung statt, bei der der Niedrigalkohol vom schwerer flüchtigen C₆- bis C₃₀-Hydrocarbylalkohol im Molekül verdrängt wird. Hierbei können neben den Umesterungsreaktionen an den Carboxylfunktionen in M1 auch Umesterungsreaktionen an der Carboxylfunktion in M2 stattfinden. Liegen die Carboxylfunktionen in M1 als freie Carbonsäuren, als Anhydride oder als Carboxylhalogenide vor, wird eine Umesterung an der Carboxylfunktion in M2 weitgehend vermieden.

Zur Veresterung oder Umesterung des Polymerisationsproduktes setzt man meist soviel an C₆- bis C₃₀-Hydrocarbylalkohol ein, dass eine oder dass beide der aus M 1 stammenden Carboxylfunktionen in langkettige Ester umgewandelt werden. Man kann die Menge an C₆- bis C₃₀-Hydrocarbylalkohol aber auch so steuern, dass dieser Veresterungs- oder Umesterungsgrad an der Carboxylfunktionen an der Wiederholungseinheit W1 zwischen 1 und 2 liegt. Wird oder soll die Carboxylesterfunktion an der Wiederholungseinheit W2 ebenfalls zu einem langkettigen Ester umgeestert werden, sind mehr als 2 Mol an C₆- bis C₃₀-Hydrocarbylalkohol pro Mol M1 notwendig, beispielsweise bis zu 3 Mol pro Mol M1, wenn M1 und M2 in äquimolarem Verhältnis copolymerisiert werden.

Die noch nicht mit dem C₆- bis C₃₀-Hydrocarbylalkohol umgesetzte, aus der radikalischen Copolymerisation der Monomereinheiten M1 und M2 resultierende Vorstufe des erfindungsgemäßen Copolymerisates weist vorzugsweise ein zahlenmittleres Molekulargewicht (Mₙ) im Bereich von 500 bis 10.000, insbesondere von 1000 bis 5000, oder alternativ ein gewichtsmittleres Molekulargewicht (M_{w}) von 750 bis 50.000, insbesondere von 1500 bis 25.000, auf (alle Angaben in g/mol, jeweils bestimmt durch Gelpermeationschromatographie).

Bezogen auf die Anzahl der Wiederholungseinheiten der Struktur W1 und W2 enthält das erfindungsgemäße Copolymerisat vorzugsweise in Summe 4 bis 80, insbesondere 8 bis 40, dieser Wiederholungseinheiten, wobei in der bevorzugten Ausführungsform des alternierenden Aufbaues des Copolymerisates die Anzahl von W1 und W2 jeweils gleich oder annähernd gleich ist.

Das erfindungsgemäße Copolymerisat dient als neuer effizienter Kaltfließverbesserer in Mitteldestillat-Kraftstoffen. Unter Mitteldestillat-Kraftstoffen sollen im Rahmen der vorliegenden Erfindung im Bereich von 120 bis 450°C siedende Mitteldestillat-Kraftstoffe verstanden werden. Solche Mitteldestillat-Kraftstoffe werden insbesondere als Dieselkraftstoff, Heizöl oder Kerosin verwendet, wobei Dieselkraftstoff und Heizöl besonders bevorzugt sind.

Mit Mitteldestillat-Kraftstoffen (nachfolgend auch kurz "Mitteldestillate" genannt) werden Kraft- und Brennstoffe bezeichnet, die durch Destillation von Rohöl als erstem Verfahrensschritt gewonnen werden und im Bereich von 120 bis 450°C sieden. Vorzugsweise werden schwefelarme Mitteldestillate verwendet, d.h. solche, die weniger als 350 ppm Schwefel, insbesondere weniger als 200 ppm Schwefel, vor allem weniger als 50 ppm Schwefel enthalten. In speziellen Fällen enthalten sie weniger als 10 ppm Schwefel, diese Mitteldestillate werden auch als "schwefelfrei" bezeichnet. Es handelt sich dabei im allgemeinen um Rohöldestillate, die einer hydrierenden Raffination unterworfen wurden, und daher nur geringe Anteile an polyaromatischen und polaren Verbindungen enthalten. Vorzugsweise handelt es sich um solche Mitteldestillate, die 90%-Destillationspunkte unter 370°C, insbesondere unter 360°C und in Spezialfällen unter 330°C aufweisen.

Schwefelarme und schwefelfreie Mitteldestillate können auch aus schwereren Erdölfraktionen gewonnen werden, die nicht mehr unter Atmosphärendruck destilliert werden können. Als typische Konversionsverfahren zur Herstellung von Mitteldestillaten aus schweren Erdölfraktionen seien genannt: Hydrocracken, thermisches Cracken, katalytisches Cracken, Cokerprozesse und/oder Visbreaking. Je nach Verfahrensdurchführung fallen diese Mitteldestillate schwefelarm oder schwefelfrei an oder werden einer hydrierenden Raffination unterworfen.

Vorzugsweise haben die Mitteldestillate Aromatengehalte von unter 28 Gew.-%, insbesondere unter 20 Gew.-%. Der Gehalt an Normalparaffinen beträgt zwischen 5% und 50 Gew.-%, vorzugsweise liegt er zwischen 10 und 35 Gew.%.

Im Sinne der vorliegenden Erfindung sollen hier unter Mitteldestillat-Kraftstoffen auch solche Kraft- oder Brennstoffe verstanden werden, welche sich entweder indirekt von fossilen Quellen wie Erdöl oder Erdgas ableiten lassen oder aber aus Biomasse über Vergasung und anschließende Hydrierung hergestellt werden. Ein typisches Beispiel für einen sich indirekt von fossilen Quellen ableitenden Mitteldestillat-Kraftstoff ist der mittels Fischer-Tropsch-Synthese erzeugte GTL("gas-to-liquid")-Dieselkraftstoff. Aus Biomasse wird beispielweise über den BTL("biomass-to-liquid")-Prozess ein Mitteldestillat hergestellt, das entweder allein oder in Mischung mit anderen Mitteldestillaten als Kraft- oder Brennstoff verwendet werden kann. Zu den Mitteldestillaten gehören auch Kohlenwasserstoffe, die durch Hydrierung von Fetten und Fettölen gewonnen werden. Sie enthalten überwiegend n-Paraffine.

Die Qualitäten der Heizöle und Dieselkraftstoffe sind beispielsweise in DIN 51603 und EN 590 näher festgelegt (vgl. auch Ullmann's Encyclopedia of Industrial Chemistry, 5. Auflage, Band A12, S. 617 ff.).

Das erfindungsgemäße Copolymerisat kann neben seiner Verwendung in den genannten Mitteldestillat-Kraftstoffen aus fossilem, pflanzlichem oder tierischem Ursprung, die im wesentlichen Kohlenwasserstoffmischungen darstellen, auch in Mischungen aus solchen Mitteldestillaten mit Biobrennstoffölen (Biodiesel) oder in reinen Biobrennstoffölen zur Verbesserung des Kaltfließverhaltens eingesetzt werden. Derartige Mischungen und auch reine Biobrennstofföle, die alle handelsüblich sind, werden im Sinne der vorliegenden Erfindung auch von dem Begriff "Mitteldestillat-Kraftstoff" umfasst. Die genannten Mischungen können die Biobrennstofföle in untergeordneten Mengen, dann typischerweise in Mengen von 1 bis 30 Gew.-% insbesondere von 3 bis 10 Gew.-%, bezogen auf die Gesamtmenge aus Mitteldestillat fossilen, pflanzlichem oder tierischen Ursprungs und Biobrennstofföl, oder in höheren Mengen bis zu 100 Gew.-% enthalten.

In einer bevorzugten Ausführungsform verwendet man das erfindungsgemäße Copolymerisat als Zusatz zu Kraftstoffen, welche aus Biobrennstoffölen (A), welche auf Fettsäureestern basieren, oder aus Mischungen aus
(A) 30 bis weniger als 100 Gew.-% mindestens eines Biobrennstofföls, welches auf Fettsäureestern basiert, und
(B) mehr als 0 bis 70 Gew.-% Mitteldestillaten aus fossilem Ursprung und/oder aus aus pflanzlichem und/oder tierischem Ursprung, welche im wesentlichen Kohlenwasserstoffmischungen darstellen und frei von Fettsäureestern sind,
bestehen.

Biobrennstofföle basieren in der Regel auf Alkylestern von Fettsäuren, die sich von pflanzlichen und/oder tierischen Ölen und/oder Fetten ableiten. Unter Alkylestern werden üblicherweise Niedrigalkylester, insbesondere C₁- bis C₄-Alkylester, verstanden, die durch Umesterung der in pflanzlichen und/oder tierischen Ölen und/oder Fetten vorkommenden Glyceride, insbesondere Triglyceride, mittels Niedrigalkoholen, beispielsweise Ethanol oder vor allem Methanol ("FAME"), erhältlich sind. Typische Niedrigalkylester auf Basis von pflanzlichen und/oder tierischen Ölen und/oder Fetten, die als Biobrennstofföl oder Komponenten hierfür Verwendung finden, sind beispielsweise Sonnenblumenmethylester, Palmölmethylester ("PME"), Sojaölmethylester ("SME") und insbesondere Rapsölmethylester ("RME").

Das erfindungsgemäße Copolymerisat bewirkt eine deutliche Verbesserung des Kaltfließverhaltens des Mitteldestillat-Kraftstoffes bzw. der Mitteldestillat-Biobrennstofföl-Mischung, d.h. eine Erniedrigung insbesondere des PP-Wertes, aber auch der CFPP- und/oder der CP-Werte, weitgehend unabhängig von der Herkunft oder der Zusammensetzung des Kraft- oder Brennstoffes. Insbesondere bei reinen Biobrennstoffölen (Biodiesel) macht sich dieser Effekt deutlich bemerkbar. Die ausgeschiedenen Paraffinkristalle werden in der Regel wirksamer in der Schwebe gehalten, so dass es nicht zu Verstopfungen von Filtern und Leitungen durch solche Sedimente kommt. Das erfindungsgemäße Terpolymerisat weist in den meisten Fällen eine gute Breitenwirkung auf und bewirkt so, dass die ausgeschiedenen Paraffinkristalle in den unterschiedlichsten Kraft- bzw. Brennstoffen sehr gut dispergiert werden.

Ebenso können durch die Verwendung des erfindungsgemäßen Copolymerisates neben der Verbesserung der Kaltfließeigenschaften von Mitteldestillat-Kraftstoffen und des Handlings mit Kaltfließverbesserer-Additiven bzw. mit Kaltfließverbesserer-Additiven enthaltenden Mitteldestillaten, beispielsweise der Verbesserung der Filtrierbarkeit der Kraftstoffe, eine Reihe weitere Kraft- bzw. Brennstoffeigenschaften verbessert werden. Exemplarisch sollen hier nur die zusätzliche Wirkung als Korrosionsschutz oder die Verbesserung der Oxidationsstabilität genannt werden.

Gegenstand der vorliegenden Erfindung sind auch Mitteldestillat-Kraftstoffe, welche 10 bis 5000 Gew.-ppm, insbesondere 25 bis 2000 Gew.-ppm, vor allem 50 bis 1000 Gew.-ppm, des erfindungsgemäßen Copolymerisates enthalten.

Die genannten Mitteldestillat-Kraftstoffe können als weitere Zusätze in hierfür üblichen Mengen noch weitere Kaltfließverbesserer, Paraffindispergatoren, Leitfähigkeitsverbesserer, Korrosionsschutzadditive, Lubricity-Additive, Antioxidantien, Metall-Deaktivatoren, Antischaummittel, Demulgatoren, Detergentien, Cetanzahl-Verbesserer, Lösungs- oder Verdünnungsmittel, Farbstoffe oder Duftstoffe oder Gemische davon enthalten. Weitere Kaltfließverbesserer sind beispielsweise in der WO 2008/113757 A1 beschrieben. Die übrigen vorstehend genannten weiteren Zusätze sind im übrigen dem Fachmann geläufig und brauchen deshalb hier nicht weiter erläutert zu werden.

Die nachfolgenden Beispiele sollen die vorliegende Erfindung erläutern, ohne sie zu beschränken.

### Beispiele

### Beispiel 1: Herstellung eines erfindungsgemäßen Copolymerisates

200 g (1,008 mol) Undec-10-ensäure-methylester wurden in 270 g Solvesso® 150 gelöst und unter Stickstoffatmosphäre auf 150°C erwärmt. Hierzu wurden innerhalb von 3 Stunden 98,9 g (1,008 mol) Maleinsäureanhydrid sowie 3,45 g Di-tert.-butylperoxid, gelöst in 30 g Solvesso® 150, bei 150°C zugegeben. Anschließend wurde noch 1 Stunde bei 150°C auspolymerisiert. Das so erhaltene Polymerisationsprodukt, welches eine streng alternierende Struktur aufwies und ein Molekulargewicht Mₙ = 1820 g/mol und M_{w} = 3520 g/mol besaß, wurde mit der 2,0-fachen molaren Menge an 1-n-Dodecanol unter Stickstoffatmosphäre für die Dauer von 6 Stunden auf 150°C erwärmt. Man erhielt eine bei Raumtemperatur gelbliche, klare, dünnviskose Flüssigkeit mit einer Säurezahl von 38,1.

### Anwendungsbeispiele 2 bis 6

Das in Beispiel 1 hergestellte Copolymerisat wurden in mehreren handelsüblichen Biobrennstoffölen auf seine Fähigkeit zur Erniedrigung des Pour Point (PP-Wert) getestet. Die nachfolgende Tabelle zeigt die nach der üblichen Methode erhaltenen Resultate der Pour Point-Bestimmungen:

| | | | |
|---|---|---|---|
| Beispiel | Biobrennstofföl | Dosierung | PP-Wert |
| Nr. | (Herkunft) | [Gew.-ppm] | [°C] |
| | | | |
| 2 | Perstorp (09/6249) RME | 0 | -12 |
| | Perstorp (09/6249) RME | 500 | <-37 |
| | | | |
| 3 | Preem (09/6234) RME | 0 | -12 |
| | Preem (09/6234) RME | 250 | <-37 |
| | Preem (09/6234) RME | 500 | <-37 |
| | | | |
| 4 | RME/SME 75/25 (08/6234) | 0 | -9 |
| | RME/SME 75/25 (08/6234) | 200 | <-37 |
| | | | |
| 5 | Perstorp (10/6134) RME | 0 | -12 |
| | Perstorp (10/6134) RME | 500 | -36 |
| | | | |
| 6 | Polen (10/6133) RME | 0 | -12 |
| | Polen (10/6133) RME | 500 | -39 |

## Patentansprüche

1. Copolymerisat, aufgebaut aus
(i) 10 bis 90 Mol-% Wiederholungseinheiten der Struktur W1 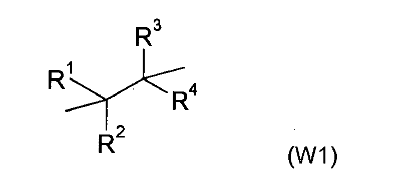 in der die Variablen R¹ und R² Wasserstoff, C₁- bis C₄-Alkyl oder Carboxylester-Gruppierungen der Formel -COOR⁹ bedeuten, wobei R⁹ für einen C₆- bis C₃₀-Hydrocarbylrest steht und wobei eine der Variablen R¹ oder R² Wasserstoff oder C₁- bis C₄-Alkyl und die andere eine Carboxylester-Gruppierung der Formel -COOR⁹ bedeutet, und
in der Variablen R³ und R⁴ Wasserstoff, C₁- bis C₄-Alkyl, Carboxylester-Gruppierungen der Formel -COOR⁹, wobei R⁹ für einen C₆- bis C₃₀-Hydrocarbylrest steht, oder Carboxylgruppen, welche auch in Form ihrer Alkali metall-, Erdalkalimetall- oder Ammoniumsalze vorliegen können, bedeuten, wobei eine der Variablen R³ oder R⁴ Wasserstoff oder C₁- bis C₄-Alkyl und die andere eine Carboxylester-Gruppierung der Formel -COOR⁹ und/oder eine Carboxylgruppe, welche auch in Form ihres Alkalimetall-, Erdalkalimetall- oder Ammoniumsalzes vorliegen kann, bedeutet, und
(ii) 90 bis 10 Mol-% Wiederholungseinheiten der Struktur W2 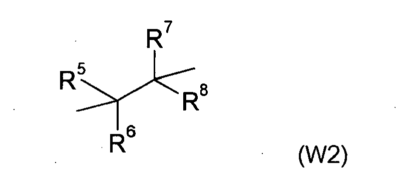 in der die Variable R⁵ den Rest eines Carbonsäureesters der Formel
-A-CO-O-R¹⁰
bedeutet, wobei die Variable A für eine C₁- bis C₂₀-Alkylengruppe steht und die Variable R¹⁰ einen C₁- bis C₃₀-Hydrocarbylrest bezeichnet, und
in der die Variablen R⁶, R⁷ und R⁸ unabhängig voneinander für Wasserstoff oder C₁- bis C₈-Alkyl stehen,
wobei die Summe der Wiederholungseinheiten W1 und W2 100 Mol-% ergibt.

2. Copolymerisat nach Anspruch 1 aus 45 bis 55 Mol-% Wiederholungseinheiten der Struktur W1 und 55 bis 45 Mol-% Wiederholungseinheiten der Struktur W2.

3. Copolymerisat nach Anspruch 1 oder 2, wobei die Variablen in der Wiederholungseinheit W1 die folgenden Bedeutungen haben:
R¹ Wasserstoff,
R² eine Carboxylester-Gruppierung der Formel -COOR⁹, wobei R⁹ für einen C₈- bis C₁₆-Hydrocarbylrest steht,
R³ Wasserstoff,
R⁴ eine Carboxylester-Gruppierung der Formel -COOR⁹, wobei R⁹ für einen C₈- bis C₁₆-Hydrocarbylrest steht, und/oder eine Carboxylgruppe, welche auch in Form ihres Alkalimetall-, Erdalkalimetall- oder Ammoniumsalzes vorliegen kann

4. Copolymerisat nach den Ansprüchen 1 bis 3, wobei die Variablen in der Wiederholungseinheit W2 die folgenden Bedeutungen haben:
R⁵ den Rest eines Carbonsäureesters der Formel -A-CO-O-R¹⁰, wobei A eine lineare C₄- bis C₁₂-Alkylengruppe und R¹⁰ einen C₁- bis C₄-Alkylrest be zeichnet,
R⁶, R⁷ und R⁸ Wasserstoff

5. Copolymerisat gemäß den Ansprüchen 1 bis 4, erhältlich durch radikalische Copolymerisation von
(i) 10 bis 90 Mol-% Monomereinheiten der Struktur M1 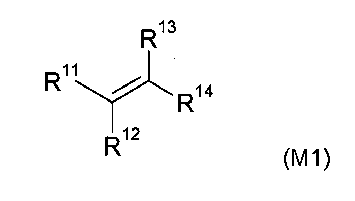 in der die Variablen R¹¹, R¹², R¹³ und R¹⁴ unabhängig voneinander Wasserstoff, C₁- bis C₄-Alkyl, Carboxylgruppen, Carboxylester-Gruppierungen der Formel -COOR¹⁵, wobei R¹⁵ für einen C₁- bis C₄-Alkylrest steht, oder Carboxylhalogenid-Gruppierungen der Formel -COX, wobei X für Fluor, Chlor, Brom oder Jod steht, bezeichnen, mit der Maßgabe, dass M1 zwei vicinal ständige derartige Carboxylgruppen und/oder Carboxylester-Gruppierungen in cis- oder trans-Stellung zueinander enthält, wobei bei vicinal ständigen Carboxylgruppen in cis-Stellung diese auch in Form ihres cyclischen Anhydrids vorliegen können, und
(ii) 90 bis 10 Mol-% Monomereinheiten der Struktur M2 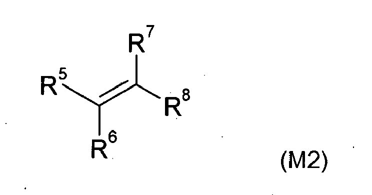 in der die Variable R⁵ den Rest eines Carbonsäureesters der Formel
-A-CO-O-R¹⁰
bedeutet, wobei die Variable A für eine C₁- bis C₂₀-Alkylengruppe steht und die Variable R¹⁰ einen C₁- bis C₃₀-Hydrocarbylrest bezeichnet, und
in der die Variablen R⁶, R⁷ und R⁸ unabhängig voneinander für Wasserstoff oder C₁- bis C₈-Alkyl stehen,
wobei die Summe der Monomereinheiten M1 und M2 100 Mol-% ergibt,
und anschließende polymeranaloge Umsetzung des gebildeten Produktes mit mindestens 1 bis 2 Mol eines C₆- bis C₃₀-Hydrocarbylalkohols pro Mol eingesetztem Monomer M1.

6. Copolymerisat nach Anspruch 5, erhältlich durch radikalische Copolymerisation von Maleinsäure, Maleinsäureanhydrid, Maleinsäuremono- oder -dimethylester, Maleinsäuremono- oder -diethylester, Fumarsäure, Fumarsäuremono- oder -dimethylester oder Fumarsäuremono- oder -diethylester als Momomereinheiten M 1.

7. Verfahren zur Herstellung eines Copolymerisates gemäß den Ansprüchen 1 bis 6, **dadurch gekennzeichnet, dass** man
(i) 10 bis 90 Mol-% Monomereinheiten der Struktur M1 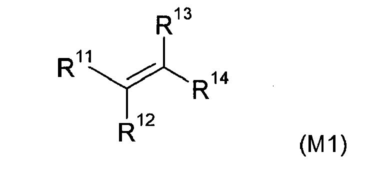 in der die Variablen R¹¹, R¹², R¹³ und R¹⁴ unabhängig voneinander Wasserstoff, C₁- bis C₄-Alkyl, Carboxylgruppen, Carboxylester-Gruppierungen der Formel -COOR¹⁵, wobei R¹⁵ für einen C₁- bis C₄-Alkylrest steht, oder Carboxylhalogenid-Gruppierungen der Formel -COX, wobei X für Fluor, Chlor, Brom oder Jod steht, bezeichnen, mit der Maßgabe, dass M1 zwei vicinal ständige derartige Carboxylgruppen und/oder Carboxylester-Gruppierungen in cis- oder trans-Stellung zueinander enthält, wobei bei vicinal ständigen Carboxylgruppen in cis-Stellung diese auch in Form ihres cyclischen Anhydrids vorliegen können, und
(ii) 90 bis 10 Mol-% Monomereinheiten der Struktur M2 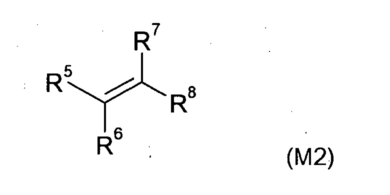 in der die Variable R⁵ den Rest eines Carbonsäureesters der Formel
-A-CO-O-R¹⁰
bedeutet, wobei die Variable A für eine C₁- bis C₂₀-Alkylengruppe steht und die Variable R¹⁰ einen C₁- bis C₃₀-Hydrocarbylrest bezeichnet, und
in der die Variablen R⁶, R⁷ und R⁸ unabhängig voneinander für Wasserstoff oder C₁- bis C₈-Alkyl stehen,
wobei die Summe der Monomereinheiten M1 und M2 100 Mol-% ergibt,
radikalisch miteinander copolymerisiert und anschließend das gebildete Produkt polymeranalog mit mindestens 1 bis 2 Mol eines C₆- bis C₃₀-Hydrocarbylalkohols pro Mol eingesetztem Monomer M1 umsetzt.

8. Mitteldestillat-Kraftstoffe enthaltend 10 bis 5000 Gew.-ppm eines Copolymerisates gemäß den Ansprüchen 1 bis 6.

9. Verwendung eines Copolymerisates gemäß den Ansprüchen 1 bis 6 zur Verbesserung der Kaltfließeigenschaften von Mitteldestillat-Kraftstoffen.

10. Verwendung nach Anspruch 9 als Zusatz zu Kraftstoffen, welche aus Biobrennstoffölen (A), welche auf Fettsäureestern basieren, oder aus Mischungen aus
(A) 30 bis weniger als 100 Gew.-% mindestens eines Biobrennstofföls, welches auf Fettsäureestern basiert, und
(B) mehr als 0 bis 70 Gew.-% Mitteldestillaten aus fossilem Ursprung und/oder aus pflanzlichem und/oder tierischem Ursprung, welche im wesentlichen Kohlenwasserstoffmischungen darstellen und frei von Fettsäureestern sind,
bestehen.

11. Verwendung nach Anspruch 9 oder 10 zur Erniedrigung des PP-Wertes von Mitteldestillat-Kraftstoffen.

## Claims

1. A copolymer formed from
(i) 10 to 90 mol% of repeat units of the structure W1 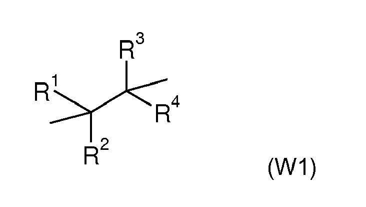 in which the variables R¹ and R² are each hydrogen, C₁- to C₄-alkyl or carboxyl ester moieties of the formula -COOR⁹ where R⁹ is a C₆- to C₃₀-hydrocarbyl radical and where one of the variables R¹ and R² is hydrogen or C₁- to C₄-alkyl and the other is a carboxyl ester moiety of the formula -COOR⁹, and
in which variables R³ and R⁴ are each hydrogen, C₁- to C₄-alkyl, carboxyl ester moieties of the formula -COOR⁹ where R⁹ is a C₆- to C₃₀-hydrocarbyl radical, or carboxyl groups which may also be present in the form of the alkali metal, alkaline earth metal or ammonium salts thereof, where one of the variables R³ and R⁴ is hydrogen or C₁- to C₄-alkyl and the other is a carboxyl ester moiety of the formula -COOR⁹ and/or a carboxyl group which may also be present in the form of the alkali metal, alkaline earth metal or ammonium salt thereof, and
(ii) 90 to 10 mol% of repeat units of the structure W2 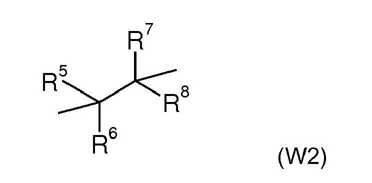
in which the variable R⁵ is the radical of a carboxylic ester of the formula
-A-CO-O-R¹⁰
where the variable A is a C₁- to C₂₀-alkylene group and the variable R¹⁰ is a C₁- to C₃₀-hydrocarbyl radical, and
in which the variables R⁶, R⁷ and R⁸ are each independently hydrogen or C₁- to C₈-alkyl,
where the sum of the repeat units W1 and W2 adds up to 100 mol%.

2. A copolymer according to claim 1 formed from 45 to 55 mol% of repeat units of the structure W1 and 55 to 45 mol% of repeat units of the structure W2.

3. A copolymer according to claim 1 or 2, wherein the variables in the repeat unit W1 are each defined as follows:
R¹ is hydrogen,
R² is a carboxyl ester moiety of the formula -COOR⁹ where R⁹ is a C₈- to C₁₆-hydrocarbyl radical,
R³ is hydrogen,
R⁴ is a carboxyl ester moiety of the formula -COOR⁹ where R⁹ is a C₈- to C₁₆-hydrocarbyl radical, and/or a carboxyl group which may also be present in the form of the alkali metal, alkaline earth metal or ammonium salt thereof.

4. A copolymer according to claims 1 to 3, wherein the variables in the repeat unit W2 are each defined as follows:
R⁵ is the radical of a carboxylic ester of the formula -A-CO-O-R¹⁰ where A is a linear C₄- to C₁₂-alkylene group and R¹⁰ is a C₁- to C₄-alkyl radical,
R⁶, R⁷ and R⁸ are each hydrogen.

5. A copolymer according to claims 1 to 4, obtainable by free-radical copolymerization of
(i) 10 to 90 mol% of monomer units of the structure M1 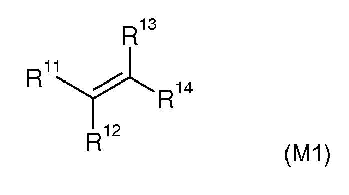 in which the variables R¹¹, R¹², R¹³ and R¹⁴ are each independently hydrogen, C₁- to C₄-alkyl, carboxyl groups, carboxyl ester moieties of the formula -COOR¹⁵ where R¹⁵ is a C₁- to C₄-alkyl radical, or carbonyl halide moieties of the formula -COX where X is fluorine, chlorine, bromine or iodine, with the proviso that M1 comprises two vicinal carboxyl groups and/or carboxyl ester moieties of this kind in cis or trans positions to one another, where vicinal carboxyl groups in cis positions may also be present in the form of the cyclic anhydride thereof, and
(ii) 90 to 10 mol% of monomer units of the structure M2 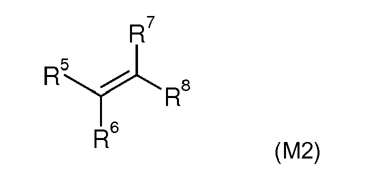 in which the variable R⁵ is the radical of a carboxylic ester of the formula
-A-CO-O-R¹⁰
where the variable A is a C₁- to C₂₀-alkylene group and the variable R¹⁰ is a C₁- to C₃₀-hydrocarbyl radical, and
in which the variables R⁶, R⁷ and R⁸ are each independently hydrogen or C₁- to C₈-alkyl,
where the sum of the monomer units M1 and M2 adds up to 100 mol%,
and subsequent polymer-analogous reaction of the product formed with at least 1 to 2 mol of a C₆- to C₃₀-hydrocarbyl alcohol per mole of monomer M1 used.

6. A copolymer according to claim 5, obtainable by free-radical copolymerization of maleic acid, maleic anhydride, mono- or dimethyl maleate, mono- or diethyl maleate, fumaric acid, mono- or dimethyl fumarate or mono- or diethyl fumarate as monomer units M1.

7. A process for preparing a copolymer according to claims 1 to 6, which comprises free-radically copolymerizing
(i) 10 to 90 mol% of monomer units of the structure M1 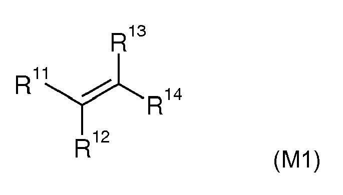 in which the variables R¹¹, R¹², R¹³ and R¹⁴ are each independently hydrogen, C₁- to C₄-alkyl, carboxyl groups, carboxyl ester moieties of the formula -COOR¹⁵ where R¹⁵ is a C₁- to C₄-alkyl radical, or carbonyl halide moieties of the formula -COX where X is fluorine, chlorine, bromine or iodine, with the proviso that M1 comprises two vicinal carboxyl groups and/or carboxyl ester moieties of this kind in cis or trans positions to one another, where vicinal carboxyl groups in cis positions may also be present in the form of the cyclic anhydride thereof, and
(ii) 90 to 10 mol% of monomer units of the structure M2 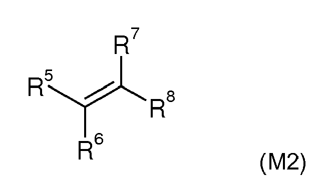 in which the variable R⁵ is the radical of a carboxylic ester of the formula
-A-CO-O-R¹⁰
where the variable A is a C₁- to C₂₀-alkylene group and the variable R¹⁰ is a C₁- to C₃₀-hydrocarbyl radical, and
in which the variables R⁶, R⁷ and R⁸ are each independently hydrogen or C₁- to C₈-alkyl,
where the sum of the monomer units M1 and M2 adds up to 100 mol%,
with one another and subsequently reacting the product formed in a polymer-analogous manner with at least 1 to 2 mol of a C₆- to C₃₀-hydrocarbyl alcohol per mole of monomer M1 used.

8. A middle distillate fuel comprising 10 to 5000 ppm by weight of a copolymer according to claims 1 to 6.

9. The use of a copolymer according to claims 1 to 6 for improving the cold flow properties of middle distillate fuels.

10. The use according to claim 9 as an additive to fuels which consist of biofuel oils (A) based on fatty acid esters, or of mixtures of
(A) 30 to less than 100% by weight of at least one biofuel oil based on fatty acid esters, and
(B) more than 0 to 70% by weight of middle distillates of fossil origin and/or of vegetable and/or animal origin, which are essentially hydrocarbon mixtures and are free of fatty acid esters.

11. The use according to claim 9 or 10 for lowering the PP value of middle distillate fuels.

## Revendications

1. Copolymère, constitué par :
(i) 10 à 90 % en moles d'unités de répétition de la structure W1 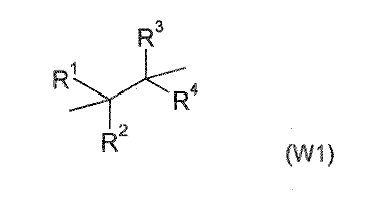 dans laquelle les variables R¹ et R² signifient l'hydrogène, un alkyle en C₁ à C₄ ou des groupes ester carboxylique de formule -COOR⁹, R⁹ représentant un radical hydrocarbyle en C₆ à C₃₀, et une des variables R¹ ou R² signifiant l'hydrogène ou un alkyle en C₁ à C₄, et l'autre signifiant un groupe ester carboxylique de formule -COOR⁹, et
dans laquelle les variables R³ et R⁴ signifient l'hydrogène, un alkyle en C₁ à C₄ , des groupes ester carboxylique de formule -COOR⁹, R⁹ représentant un radical hydrocarbyle en C₆ à C₃₀, ou des groupes carboxyle, qui peuvent également se présenter sous la forme de leurs sels de métaux alcalins, de métaux alcalino-terreux ou d'ammonium, une des variables R³ ou R⁴ signifiant l'hydrogène ou un alkyle en C₁ à C₄ , et l'autre signifiant un groupe ester carboxylique de formule -COOR⁹ et/ou un groupe carboxyle qui peut également se présenter sous la forme de son sel de métal alcalin, de métal alcalino-terreux ou d'ammonium, et
(ii) 90 à 10 % en moles d'unités de répétition de la structure W2 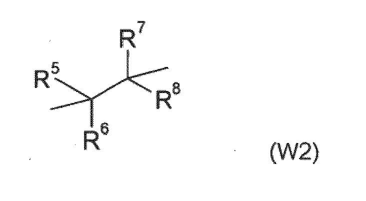
dans laquelle la variable R⁵ signifie le radical d'un ester d'acide carboxylique de formule
-A-CO-O-R¹⁰
la variable A représentant un groupe alkylène en C₁ à C₂₀ et la variable R¹⁰ désignant un radical hydrocarbyle en C₁ à C₃₀, et
dans laquelle les variables R⁶, R⁷ et R⁸ représentent indépendamment les unes des autres l'hydrogène ou un alkyle en C₁ à C₈,
la somme des unités de répétition W1 et W2 étant de 100 % en moles.

2. Copolymère selon la revendication 1, constitué de 45 à 55 % en moles d'unités de répétition de la structure W1 et de 55 à 45 % en moles d'unités de répétition de la structure W2.

3. Copolymère selon la revendication 1 ou 2, dans lequel les variables dans l'unité de répétition W1 ont les significations suivantes :
R¹ l'hydrogène,
R² un groupe ester carboxylique de formule -COOR⁹, R⁹ représentant un radical hydrocarbyle en C₈ à C₁₆,
R³ l'hydrogène,
R⁴ un groupe ester carboxylique de formule -COOR⁹, R⁹ représentant un radical hydrocarbyle en C₈ à C₁₆, et/ou un groupe carboxyle, qui peut également se présenter sous la forme de son sel de métal alcalin, de métal alcalino-terreux ou d'ammonium.

4. Copolymère selon les revendications 1 à 3, dans lequel les variables dans l'unité de répétition W2 ont les significations suivantes :
R⁵ le radical d'un ester d'acide carboxylique de formule -A-CO-O-R¹⁰, A désignant un groupe alkylène en C₄ à C₁₂ linéaire et R¹⁰ un radical alkyle en C₁ à C₄,
R⁶, R⁷ et R⁸ l'hydrogène.

5. Copolymère selon les revendications 1 à 4, pouvant être obtenu par copolymérisation radicalaire de
(i) 10 à 90 % en moles d'unités monomères de structure M1 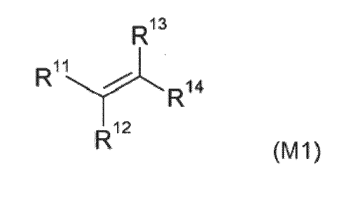 dans laquelle les variables R¹¹, R¹², R¹³ et R¹⁴ désignent indépendamment les unes des autres l'hydrogène, un alkyle en C₁ à C₄ , des groupes carboxyle, des groupes ester carboxylique de formule -COOR¹⁵, R¹⁵ représentant un radical alkyle en C₁ à C₄, ou des groupes halogénure de carboxyle de formule -COX, X représentant fluor, chlore, brome ou iode, à condition que M1 contienne deux tels groupes carboxyle et/ou groupes ester carboxylique vicinaux en position cis ou trans l'un par rapport à l'autre, en cas de groupes carboxyle vicinaux en position cis, ceux-ci pouvant également se présenter sous la forme de leur anhydride cyclique, et
(ii) 90 à 10 % en moles d'unités monomères de structure M2 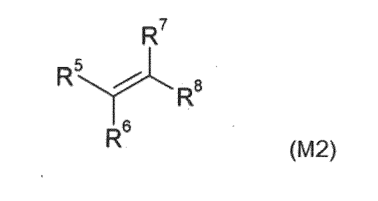
dans laquelle la variable R⁵ signifie le radical d'un ester d'acide carboxylique de formule
-A-CO-O-R¹⁰
la variable A représentant un groupe alkylène en C₁ à C₂₀ et la variable R¹⁰ désignant un radical hydrocarbyle en C₁ à C₃₀, et
dans laquelle les variables R⁶, R⁷ et R⁸ représentent indépendamment les unes des autres l'hydrogène ou un alkyle en C₁ à C₈,
la somme des unités monomères M1 et M2 étant de 100 % en moles,
puis réaction polymère-analogue du produit formé avec au moins 1 à 2 moles d'un alcool hydrocarbylique en C₆ à C₃₀ par mole de monomère M1 utilisé.

6. Copolymère selon la revendication 5, pouvant être obtenu par copolymérisation radicalaire d'acide maléique, d'anhydride de l'acide maléique, d'ester mono- ou diméthylique de l'acide maléique, d'ester mono- ou diéthylique de l'acide maléique, d'acide fumarique, d'ester mono- ou diméthylique de l'acide fumarique ou d'ester mono- ou diéthylique de l'acide fumarique en tant qu'unités monomères M1.

7. Procédé de fabrication d'un copolymère selon les revendications 1 à 6, **caractérisé en ce que**
(i) 10 à 90 % en moles d'unités monomères de structure M1 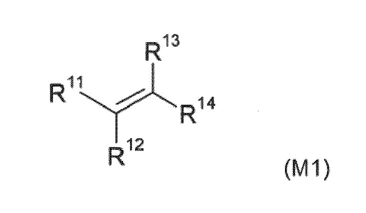 dans laquelle les variables R¹¹, R¹², R¹³ et R¹⁴ désignent indépendamment les unes des autres l'hydrogène, un alkyle en C₁ à C₄ , des groupes carboxyle, des groupes ester carboxylique de formule -COOR¹⁵, R¹⁵ représentant un radical alkyle en C₁ à C₄, ou des groupes halogénure de carboxyle de formule -COX, X représentant fluor, chlore, brome ou iode, à condition que M1 contienne deux tels groupes carboxyle et/ou groupes ester carboxylique vicinaux en position cis ou trans l'un par rapport à l'autre, en cas de groupes carboxyle vicinaux en position cis, ceux-ci pouvant également se présenter sous la forme de leur anhydride cyclique, et
(ii) 90 à 10 % en moles d'unités monomères de structure M2 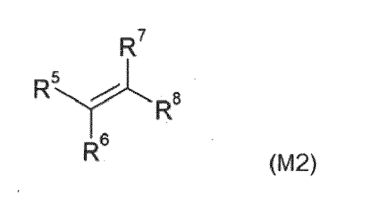
dans laquelle la variable R⁵ signifie le radical d'un ester d'acide carboxylique de formule
-A-CO-O-R¹⁰
la variable A représentant un groupe alkylène en C₁ à C₂₀ et la variable R¹⁰ désignant un radical hydrocarbyle en C₁ à C₃₀, et
dans laquelle les variables R⁶, R⁷ et R⁸ représentent indépendamment les uns des autres l'hydrogène ou un alkyle en C₁ à C₈,
la somme des unités monomères M1 et M2 étant de 100 % en moles,
sont copolymérisées par voie radicalaire les unes avec les autres, puis le produit formé est mis en réaction polymère-analogue avec au moins 1 à 2 moles d'un alcool hydrocarbylique en C₆ à C₃₀ par mole de monomère M1 utilisé.

8. Carburants à base de distillats moyens contenant 10 à 5 000 ppm en poids d'un copolymère selon les revendications 1 à 6.

9. Utilisation d'un copolymère selon les revendications 1 à 6 pour améliorer les propriétés d'écoulement à froid de carburants à base de distillats moyens.

10. Utilisation selon la revendication 9 en tant qu'additif pour carburants constitués de biocarburants huileux (A) à base d'esters d'acides gras, ou de mélanges de
(A) 30 à moins de 100 % en poids d'au moins un biocarburant huileux à base d'esters d'acides gras et
(B) plus de 0 à 70 % en poids de distillats moyens d'origine fossile et/ou d'origine végétale et/ou animale qui sont essentiellement des mélanges d'hydrocarbures et sont exempts d'esters d'acides gras.

11. Utilisation selon la revendication 9 ou 10 pour réduire la valeur PP de carburants à base de distillats moyens.
